# EUROPEAN PATENT APPLICATION

(11) **EP 1 312 353 A1**
(43) Date of publication of application: **21.05.2003**
(21) Application number: 01811103.9
(22) Date of filing: 16.11.2001
(51) Int. Cl.: A61K 7/155

(54) **Method for hair removal**

(71) Applicant: ECOLE POLYTECHNIQUE FEDERALE DE LAUSANNE (EPFL), 1015 Lausanne (CH)
(72) Inventor: Wagnières, Georges, Dr., 1095 Lutry (CH); Lange, Norbert, Dr., 1004 Lausanne (CH); Dögnitz, Nora, Dr., 1004 Lausanne (CH); Salomon, Denis, Dr., 1213 Petit-Lancy (CH); van den Bergh, Hubert, Prof., 1025 St-Sulpice (CH)
(74) Representative: Ganguillet, Cyril

(57) **Abstract**

A method for hair removal from a skin area by selective photo-inactivation of the pilo-sebaceous apparatus using derivatives of ALA with lipophylic chains. Selectivity of the method is further enhanced by preliminary treatment of the epidermis or by agents reducing PpIX levels in the epidermis.

## Description

The present invention concerns a cosmetic method for hair removal.

Currently, the most common methods for hair removal involve the use of hair removal creams, as well as shaving, waxing and electrolysis. Although creams and shaving are popular because they can be readily used at home, they are inadequate because they must be used on a very regular basis. Electrolysis offers longer-term hair removal. This method, however, can be time-consuming, is often quite painful and expensive.

Lasers, lamps and other sources of electromagnetic radiation are being increasingly utilized for the removal of unwanted hair, and for at least inhibiting, and in some instance preventing, the regrowth thereof. Examples of such epilation techniques are disclosed by U.S. 5,227,907 and U.S. 5,425,728 (to Tankovich), describing topical formulations containing a substance having high absorption at a frequency band of light, and capable to infiltrate a hair duct. Such substances may be carbon particles, hematoporphyrin and/or various dyes. The formulation is applied to the skin, the excess is removed and the skin is illuminated with an appropriate light source so that the energy absorbed is sufficient to cause reactions which destroy hairs or ducts or follicles or tissues surrounding said follicles. Such treatments are generally not satisfactorily selective, in that they damage surrounding tissues instead of the hair follicle itself, and may provoke adverse skin reactions.

U.S. 5,669,916 and U.S. 5,989,267 (to Anderson) feature a method involving mechanically or chemically removing of the hair to expose the follicle and then treating topically the follicle by an inactivating compound to inhibit its ability to regenerate a hair. The preliminary removing of the hair facilitates the uptake of the follicle-inactivating compound via the hair duct. The follicle-inactivating compound may be a dye or a photosensitizer, filling the empty follicle, which is submitted thereafter to a light exposure with sufficient energy and for sufficient duration to destroy the follicle. U.S. 5,669,916 discloses the use of aminolevulinic acid (ALA), a photosensitizer precursor of protoporphyrin IX (PpIX), a naturally occurring photosensitizer, which is the immediate precursor of Heme in the Heme bio-synthetic pathway and which may be synthesized in relatively large quantities by certain cells in the presence of ALA. When ALA is administered, the exposition to a light is delayed by several hours for allowing synthesis of PpIX.

A drawback of this method is that in a given area of skin all hair follicles are not in same physiological state, that is to say, do not bear a hair at the same time, and thereby cannot be depilated simultaneously. Thus, the Anderson method, unless repeated several times, leads only to the inactivation of a fraction of the follicles present in the treated area.

It is on the other hand known that cutaneous administration of ALA results in a localization of ALA predominantly in the superficial skin layers, with a relatively low selectivity versus cell types, and thereby induces a similarly spread synthesis of PpIX, so that a subsequent irradiation with visible light results in damage of various epidermal cell types and tissues.

Accordingly, there exists a need for a method for selectively removing hair, that is cheap, that is not time-consuming, painful and damaging to the skin, and results in hair removal which is long lasting and more permanent than known hair removal methods.

These objects are achieved by a cosmetic method for removing hair from a selected skin area comprising the use of a compound of formula (I)

R²R³N-CH₂COCH₂-CH₂COOR¹ (I)

wherein R¹, R², R³ represent each separately H, an unsubstituted alkyl, or a substituted alkyl, wherein suitable substitutents are aryl, acyl, halogen, hydroxy, amino, aminoalkyl, alkoxy, acylamino, thioamino, acyloxy, aryloxy, aryloxyalkyl, mercapto, thia, azo, oxo groups, saturated and non-saturated cyclohydrocarbons, and heterocycles, or represent an alkyl chain interrupted by oxygen, nitrogen, sulfur or phosphor atoms, or an alkoxycarbonyloxy, alkoxycarbonylalkyl or methine group, and wherein R¹, R² and R³ do not represent simultaneously H, for photoinactivating hair follicles of said skin area.

The inventors have found that the substitution of at least one H of either the carboxyl or the amino group of ALA by a more lipophilic substituant provides an unexpectedly high and selective accumulation of PpIX in hair follicles and sebaceous glands, if compared to the application of ALA itself as depicted above. The phenomena increases within a time span of about 20 hours after the beginning of a topical administration of the photosensitizer precursor substance to the treated skin area.

The use of ALA esters (R² = R³ = H) or pharmaceutically acceptable salts thereof where the group R¹ is an alkyl from C1 to C12 is preferred. Particularly preferred photosensitizer precursors are n-hexyl-ALA and n-octyl-ALA.

The photosensitizer precursor may be administered parenterally, in particular by subcutaneous injection. Preferably, the photosensitizer precursor is administered topically to the skin area to be treated.

Preferred embodiments, pertaining either to the technique of administration or to the formulation comprising the administered photosensitizer precursor, or to both, increase selectivity by:
- minimizing uptake of the photosensitizer precursor in the epidermis;
- minimizing biosynthesis of PpIX in the epidermis;
- maximizing catabolism of PpIX in the epidermis;
- minimizing phototoxic effect of light exposure in the epidermis;
- maximizing selective uptake of the photosensitizer precursor by the pilo-sebaceous apparatus;
- maximizing biosynthesis of PpIX in the pilo-sebaceous apparatus;
- slowing down catabolism of PpIX in the pilo-sebaceous apparatus;
- enhancing phototoxic effect of light exposure in the pilo-sebaceous apparatus.

The method of the invention may employ iontophoretic delivery techniques for overcoming the skin barriers and in particular the stratum corneum.

The method may involve the removal of the stratum corneum for enhancing penetration of a component of the formulation.

Since the bio-synthesis of PpIX is strongly temperature dependent, in an embodiment of the method of the invention, the superficial layers of the skin are cooled from outside, so that bio-synthesis of PpIX is diminished in these superficial epidermal layers but not in the deeper dermal layers.

Since the phototoxicity of PpIX is increased in the presence of oxygen, according to another embodiment of the method of the invention, the area of skin to be treated is maintained under a medium free of oxygen, thereby depleting the superficial layers from oxygen.

The method of the invention may employ a specific sequence of light irradiations, comprising at least a first irradiation performed with poorly penetrating wavelengths, that is to say smaller than 600 nm, preferably around 400 nm, and with a high light flux. The first irradiation is followed by at least a second irradiation performed with light having a more penetrating wavelength, above 600 nm, preferably with red light around 635 nm, and with a sufficient total dose, but at a sufficiently small light flux to damage the pilo-sebaceous apparatus, while sparing the epidermis in which the PpIX has been degraded with the first irradiation. Having examined the effects of light dose and light flux on the photo-induced damage of skin portions having received ALA or ALA derivatives, the inventors have found that the photo-induced damage does not depend solely on the total light dose, but depends also on the light flux and the initial concentration of sensitizer. For a given total dose, the photo-induced damage increases with decreasing light flux. Also, at high light fluxes of 50 - 150 mW/cm², at 514 nm, oxygen depletion is found to have a significant effect. Therefore, a selective destruction of PpIX in the epidermis can be achieved by irradiating the skin at high light flux and with a poorly penetrating wavelength. This irradiation will degrade the PpIX in the epidermis while generating minimal tissue destruction in this tissue layer. The PpIX located in the pilo-sebaceous apparatus will not be significantly excited, due to the poor penetration of the wavelength mentioned above. A subsequent irradiation of the skin with a longer, more penetrating wavelength, and with a sufficiently small light flux damages then the pilo-sebaceous apparatus while sparing the epidermis, in which the PpIX has been degraded with the first irradiation.

According to another embodiment of the method of the invention, the phototoxic effects induced by the irradiation can be selectively reduced in the epidermis by administering topically antioxidants or free radical scavengers, or substances reacting with singlet oxygen. Examples of such substances are vitamin B6, C, ascorbic acid, E (tocopherols) and derivatives thereof (ester), vitamin A and carotenoids (alpha, beta and gamma-carotene, lycopene, lutein, etc.), retinoids, azides, superoxyde-dismutase, butyl-hydroxytoluene, 1,4-diazabicyclo [2,2,2] octane, histidin, L-tryptophan, n-acetyl-1-cysteine, 1-cysteine, s-adenosyl-1-methionine, melatonin, 1-melatonin, DHEA or other hormones with antioxidant activity, glycine, mannitol, reduced or non-reduced glutathione, Se-glutathione peroxidase, Fe-catalase, NADPH, ubiquinol (reduced coenzyme Q10), Zn-superoxide dismutase (SOD), Mn-SOD, Cu-SOD, uric acid, lipoic acid, alpha-hydroxy acids, metal binding proteins including albumin (and albumin bound thriols and bilirubin), Fe and Cu-binding proteins (transferrin, ceruloplasmin), Fe-complex binding proteins like heptoglobin and hemopexin, metals essential for the function of antioxidant enzymes like Se, food antioxidants (BHA, BHT, propyl gallate, TBHQ, rosemary extract). A combination of some of these agents improves their efficacy.

Agents may also be used to reduce the PpIX synthesis efficacy in the epidermis. Particularly preferred additives are inhibitors of protoporphyrinogen oxidase, a mitochondrial enzyme responsible for the conversion of protoporphyrinogen to PpIX. Surprisingly, whereas publications in the agricultural and biomedical fields report an increase of PpIX production after administration of inhibitors of this enzyme, the inventors found that this additive decreases the overall PpIX production in T24 bladder cells; the mechanism is not fully understood.

Other additives are inhibiting the cellular transport of PpIX precursors, in particular 5-aminolevulinic acid methyl ester, thus potentially reducing the PpIX generation in the epidermis. Such inhibitors include: 2-deoxyglucose, sodium azide and nonpolar amino acids such as 1-alanine, 1-methionine, 1-tryptophan and glycine. Moreover, with the exception of valine, methionine and threonine, zwitterionic and basic amino acids inhibit the transport of 5-ALA. The transport of this precursor is most efficiently inhibited by transporters of the beta system, including but not limited to the beta-amino acids, beta-alanine and taurine and by gamma-aminobutyric acid (GABA). The cellular internalization of PpIX precursors can also be reduced using inhibitors of the active transport, in particular those playing a role in the ion (in particular sodium and potatium ions) migration through the cell membrane channels.

According to the present invention, metal ions, in particular iron ions, may be supplied as physiologically compatible salts or complexes thereof in a suitable concentration for reducing average PpIX level in epithelium. These compounds act, among others, as co-enzymes and bio-catalysts or as regulators of water equivalence and osmotic pressure. Beyond their essential role in tissue growth in humans, metal ions such as, in particular, iron ions are mandatory in the ferrochelatase-mediated transformation of phototoxic PpIX into phototoxically inactive heme. Therefore, the co-administration of bioavailable ferrochelatase, combined or not combined with metal salts, in particular iron salts, with the active compounds according to this invention may decrease unwanted PpIX accumulation in different skin layers, such as the epithelium, while maintaining optimal PpIX generation in the pilo-sebaceous apparatus and formulation.

Examples for such salts include, but are not limited to, oxides, chlorides, sulfates, phosphates, citrates, lactates, glycerophosphates, gluconates, edetates, tartrates, malates, mandelates, benzoates, salicylates, phytates, cinnamates, fumarates, polysaccharide complexes, or amino acid salts. Principally every salt of those elements is suitable for releasing metal atoms, in particular iron, in defined amounts in the skin care compositions according to the invention.

Alternatively, metal ions, in particular iron ions, can be provided in the form of complexes or chelates. Known watersoluble chelates of iron which are relatively or substantially non-toxic are desferrioxamine methanesulfonate, ethylenediaminetetraacetic acid (EDTA) and salts thereof, diethylenetriamine pentaacetic acid (DTPA) and salts thereof, nitrilotriacetic acid (NTA) and salts thereof, trans-1,2-diaminocyclohexane-N,N,N',N'-tetraacetic acid and salts or hydrates thereof, 1,3-diamino-2-hydroxypropyl-N,N,N',N'-tetraacetic acid and salts or hydrates thereof and ethyleneglycol-bis (beta-aminoethyl ether)-N,N-tetraacetic acid, saccharose octasulfate complexes, or 8-hydroxyquinoline complexes.

Metal ions, in particular iron ions, can be administered under the form of metal-complex binding proteins such as heptoglobin and hemopexin.

Furthermore, the cellular uptake of metal ions, in particular iron ions, can be enhanced when uptake-modulating substances such as lactoferrin, transferrin or protein analogous thereof are given alone or along with the metal salts and complexes mentioned above.

A composition containing the photosensitizer precursor according to the invention may also contain one or several agents enhancing penetration ability of the sensitizer precursor down to the hair follicle and sebaceous gland, or increase bio-synthesis of PpIX, or inhibit further biochemical steps leading from PpIX to heme. Preferred agents are DMSO, EDTA, alcohols, in particular ethanol, and deferoxamine. Deferoxamine is particularly preferred since this substance is not able to cross the stratum corneum but is able to migrate along a hair duct, and thereby increases the difference in PpIX accumulation between follicles and surrounding tissues.

The above-mentioned agents and additives may be formulated together with a compound of formula (I) for topical administration. Topical compositions include, but are not limited to solutions, gels, creams, ointments, sprays, lotions, salves, sticks, soaps, powders, pessaries, aerosols, and other conventional pharmaceutical forms in the art, which may be administered with or without occlusion.

Solutions may, for example, be formulated with an aqueous or alcoholic base containing one ore more emulsifying, surfacting, dispersing, suspending, penetration enhancing, or thickening agent.

Ointments and creams may, for example, be formulated with an aqueous or oily base with the addition of suitable thickening and/or gelling and/or surfacting agents.

Lotions may be formulated with an aqueous or oily base and will, in general, also contain one or more emulsifying, surfacting, dispersing, suspending, or thickening agent. Suitable surfacting agents are lauryl derivatives.

The compounds according to the invention may be provided in liposomal formulations. Pharmaceutically acceptable liposomal formulations are well-known to persons skilled in the art and include, but are not limited to, phosphatidyl cholines, such as dimyristoyl phosphatidyl choline (DMPC), phosphatidyl choline (PC), dipalmitoyl phosphatidyl choline (DPPC), and distearoyl phosphatidyl choline (DSP), and phosphatidyl glycerols, including dimyristoyl phosphatidyl glycerol (DMPG) and egg phosphatidyl glycerol (EPG). Such liposomes may optionally include other phospholipids, e.g. phosphatidyl ethanolamine, phosphatic acid, phosphatidyl serine, phosphatidyl inositol, abd disaccarides or poly saccarides, including lactose, trehalose, maltose, maltotriose, palatinose, lactulose, or sucrose in a ratio of about 10-20 to 0.5-6, respectively.

These additives may also conveniently be provided in a separate vial, within a kit comprising the compound of formula (I) thereby permitting sequential delivery of agents and compounds, taking into account the kinetics of PpIX synthesis in different tissues.

Synthesis of PpIX in a hair follicle is depending upon the growing state of the hair. For enhancing the efficiency of the method, most hair follicles of a treated skin area should be as far as possible in the same growing phase. The method according to the invention may thus be preceded by a preliminary synchronization step, by means of substances like minoxidil and/or by a preliminary epilation.

The authors found that a preliminary removal of the greasy and/or lipophylic substances from surface of the skin, for example by means of acetone or alcoholic solutions, enhances build up of PpIX in the pilo-sebaceous apparatus. Also, as a preliminary step, it has been found useful to submit the skin to heat and humidity, for example by washing the area to treat with warm water or by applying thereon warm wet towels.

Advantages of the use of compounds according to the invention will further appear to those skilled in the art by the following description of experimental results evidencing the selectivity of preferred compounds versus hair follicles, in relation to the drawings, wherein
- Figure 1 shows the PpIX fluorescence intensity for different PpIX precursor concentrations (h-ALA: 0.5 - 5 %, ALA: 20 %) measured by fluorescence spectroscopy on normal skin of volunteers after an application time of 6 hours.
- Figure 2 shows PpIX fluorescence kinetics measured on a normal skin of two volunteers (open and closed symbols) for PpIX precursor concentrations of 0.5 % (o), 1 % (Δ) and 1.5 % n-hexyl-ALA (◇), and 20 % ALA.
- Figure 3 shows the PpIX fluorescence distribution in a sample of excised carcinomal skin tissue by fluorescence microscopy images (a-d) and corresponding hematoxylin-eosin stained images (e-h) of skin tumors obtained from 5 µm thick tissue cross sections. a, e: squamous cell carcinoma of the head (20 % ALA, 20 hours and 30 minutes application); b, f: basocellular carcinoma (BCC) of the thorax (20 % ALA, 20 hours application); c, g: BCC of the head (1 % h-ALA, 2 hours and 20 minutes application); d, h: BCC of the thorax (1 % h-ALA, 15 hours application);
- Figure 4 shows a photomicrography of a human 5 µm skin cross section from the skin of the neck. A) Fluorescence image, B) hematoxylin-eosin stained images. Resection performed 3 hours and 30 minutes after the administration of 1 % h-ALA (5 % DMSO). e) epithelium, sc) stratum corneum, t) tumor, d) dermis, h) hair follicle.
- Figure 5 shows the anodal iontophoretic flux of PpIX presursors from aqueous solutions measured in the skin dermatomed from porcine ears.
- Figure 6 shows the PpIX fluorescence produced by T24 cells 4.5 hours after incubation with h-ALA (0.1 mM) for different concentrations of an inhibitor of protoporphyrinogen oxidase.

PpIX fluorescence in skin was induced by the topical application of an amphiphilic emulsion (Decoderm Basiscreme, Hermal Kurt Herrmann, Reinbek, Germany) containing 5 % (w/w) DMSO (Fluka, Neu-Ulm, Germany) and the PpIX precursor at the following concentrations: 20 % (w/w) ALA (ALA hydrochlorid, Merck, Darmstadt, Germany) or 0.25 %, 0.5 %, 1 %, 1.5 %, 2.5 %, 5 % n-hexyl-ALA produced according to the method described by Lange et al in Brit. J. Cancer, 80(1/2), pp 185-193, 1999. An ALA concentration of 20 % is proposed by US 5,669,916 and is frequently used in dermatology for phototherapies because PpIX fluorescence generation is poorly dose dependent for ALA applications between 10 - 30 %. The emulsions were prepared 24 hours before application and stored at 4°C in the dark until use.

For fluorescence spectroscopy, the formulations prepared as described above, and one emulsion without precursor, were applied to eight separate sites of the inner fore arms of five volunteers (two females, three males) for up to 23 hours on to a surface of 10 x 20 mm. Then the application site was protected using a transparent dressing (Tegaderm, 2M, Switzerland) and opaque tissue to avoid unwanted light exposition. Prior to measurements, the emulsions were removed.

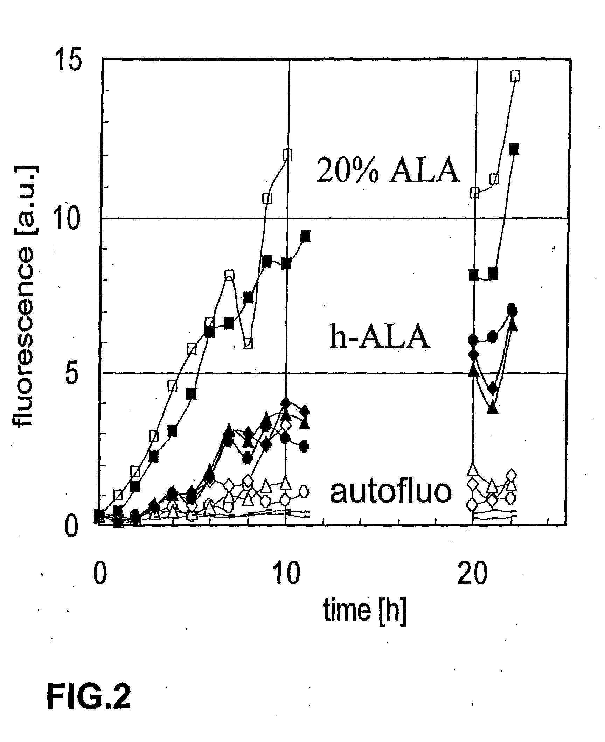

The pharmacokinetics of PpIX and the autofluorescence of normal skin were measured on the normal skin of two volunteers (full and empty symbols in Fig. 2) with an optical fiber-based fluorescence spectrofluorometer described by Braichotte et al in Cancer, 75(11), pp 2768-2778, 1995. It consists of a Xenon Lamp, an excitation monochromator, a spectrograph, an intensified diode array and an optical multichannel analyzer. Excitation and detection wavelengths were separated by a 525-nm excitation shortpass filter, 500-nm dichroic filter and a 590-nm emission longpass filter. The PpIX fluorescence was excited at 510 nm (7 nm FWHM) with a power of 20 µW at the end of the quartz fiber (diameter 600 µm, 5 m long, NA 0.4).

For each condition, four fluorescence spectroscopic measurements were performed. For this purpose, the distal end of the fiber was brought into slight contact with the skin and replaced perpendicularly on the skin. Then, the fiber tip was cleaned with ethanol before measuring a site with a different PpIX precursor concentration. Each spectrum was corrected for the background and for day-to-day fluctuations of the excitation light energy or detection pathway alignment by using a Rhodamine B ethanol solution (10⁻⁶ mol/l) in a quartz cuvette as reference.

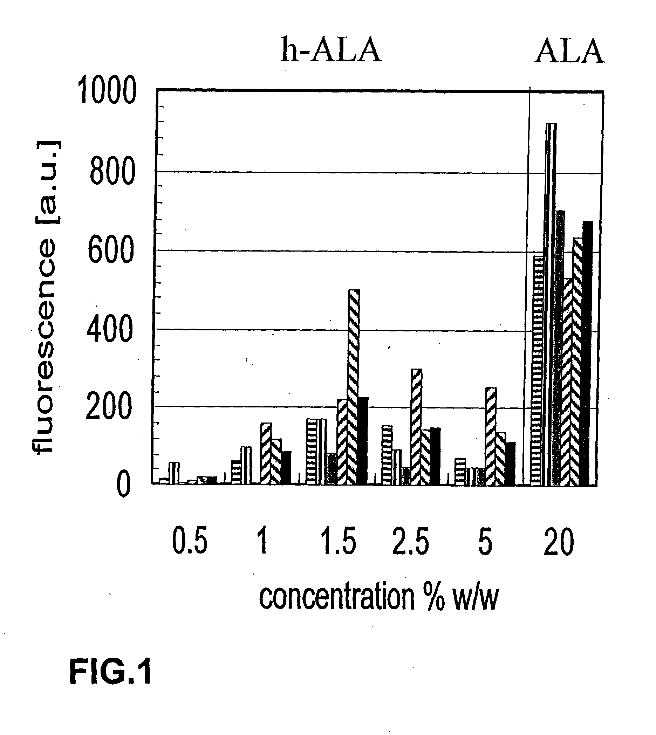

Dose-response curves for formulations containing n-hexyl-ALA concentrations ranging from 0.25 to 5 % (w/w) were established on the healthy skin of five volunteers and compared to the PpIX fluorescence intensities induced by a formulation containing 20 % (w/w) of ALA after an application time of six hours. Measurements performed on the areas exposed to the emulsion without PpIX precursor were used as autofluorescence reference. The PpIX fluorescence intensities measured for each volunteer and mean values depending on the different precursor concentrations are shown in Fig. 1. Using a concentration of n-hexyl-ALA of 0.25 %, only one volunteer showed a hardly detectable PpIX fluorescence signal after six hours of application. Therefore, these results are omitted in Fig. 1. Overall, the highest PpIX fluorescence intensities were observed when formulations containing 20 % of ALA were used. Nevertheless, they were found to exceed the highest fluorescence intensities of the ester using the optimal concentration of 1.5 %, that is more than ten times less, in average by a factor of only three.

In two volunteers, the PpIX formation was followed during 23 hours after topical administration of creams containing 0.25 %, 0.5 %, 1 % and 1.5 % of n-hexyl-ALA and 20 % of ALA. Fig. 2 shows the fluorescence time profiles observed. Under all conditions, the fluorescence intensities increased with the application time. PpIX fluorescence intensities induced by the formulation containing ALA was about three times higher as compared to those after exposure to n-hexyl-ALA during the first 10 hours. ALA application times longer than 20 hours did not further increase substantially the PpIX accumulation as compared to 10 hours of continuous application. On the contrary, application times longer than 20 hours increased the fluorescence intensity for all concentrations of n-hexyl-ALA by a factor of about 1,6. Thus, after 20 hours, the ALA induced fluorescence intensity is about twice the n-hexyl-ALA induced fluorescence, whereas ALA is applied in a concentration about 20 times the concentration of n-hexyl-ALA.

Fluorescence microscopy of PpIX distribution following exposure to ALA and n-hexyl-ALA on excised tissue samples and human skin lesions of a group of patients necessitating a treatment by surgical excision, was carried out employing a quadro-ocular epi-fluorescence microscope (Labophot-2, Nikon, Japan) comprising a mercury vapor lamp HbO 100W, a microscopy objective Plan Fluor 10/0.30 (Nikon, Japan), and a Nikon Cube BV2A (excitation filter 400-440 nm, dichroic mirror 455 nm, barrier filter 470 nm). PpIX fluorescence and autofluorescence were detected with a longpass filter 610 nm (RG610, Schott, Germany) and a band-pass filter 550 ± 20 nm (550FS40-25, Andover, NH, USA), respectively.

Fluorescence microscopy images were detected with a 12-16 bits-ST-133-MicroMax controller (Princeton Instruments, NJ, USA) connected to a cooled CCD-chip camera (Kodak KAF1600) and employing IPLAB Spectrum image processing and camera driving software (Signal Analytics Corp., Virginia, USA). A 1,02 mm thick ruby plate (8 Sp3, Hans Stettler S.A., Lyss, Switzerland) served as fluorescence reference. A fluorescence reference image and a background image were recorded before and after acquisition of the tissue fluorescence images. Usually, a series of three images consisting of (i) A PpIX fluorescence image detected via the red longpass filter; (ii) an autofluorescence image via the green bandpass filter; and (iii) a normal microscopic image after staining with toluidin blue were taken for each tissue cross section. The exposure times for fluorescence excitation light were hold to two seconds and caused less than 10 % PpIX fluorescence bleaching (data not shown).

Image processing was performed as follows: the background image was subtracted from the tissue fluorescence images and from the ruby reference image. Then, the PpIX fluorescence image was corrected for autofluorescence by subtracting the autofluorescence image multiplied by a correction factor of 2.7, which was determined from images obtained from tissue cross sections of three excised BCCs without application of a PpIX precursor. It was calculated as the ratio of the BCC autofluorescence via the red detection filter and via the green band pass filter. The resulting PpIX fluorescence image, corrected for background and autofluorescence, was normalized by the ruby reference image to correct for day-to-day fluctuations of the excitation light energy and for illumination inhomogeneities.

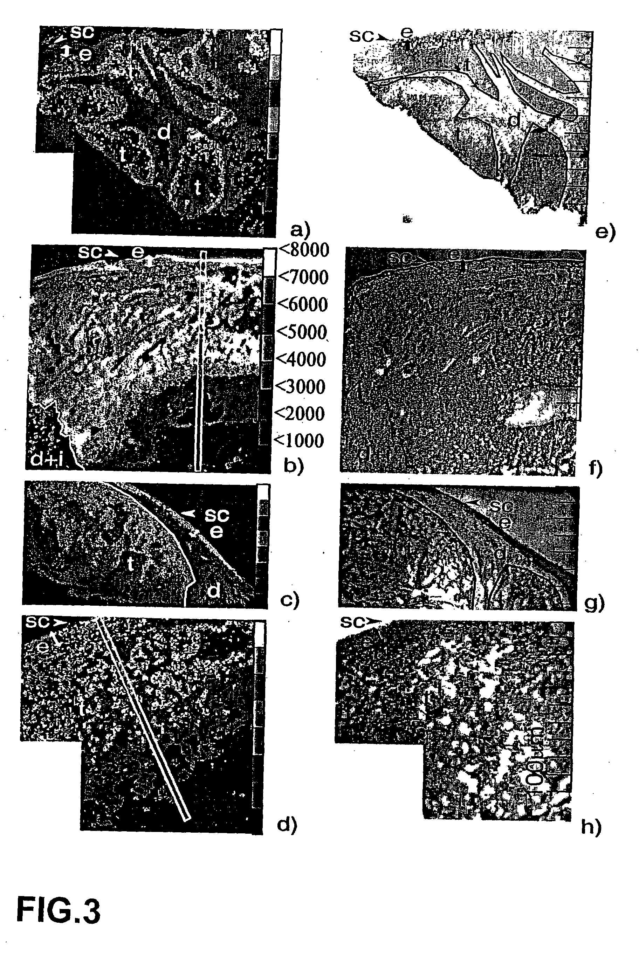

In the fluorescence micrographs presented in Fig. 3, PpIX can be observed predominantly in tumor tissue, the epidermis as well as inflamed areas, giving a good fluorescence contrast to the dermis. The fluorescence intensities are coded using a linear scale and can be compared quantitatively, because images were corrected for background and autofluorescence and normalized with respect to a reference.

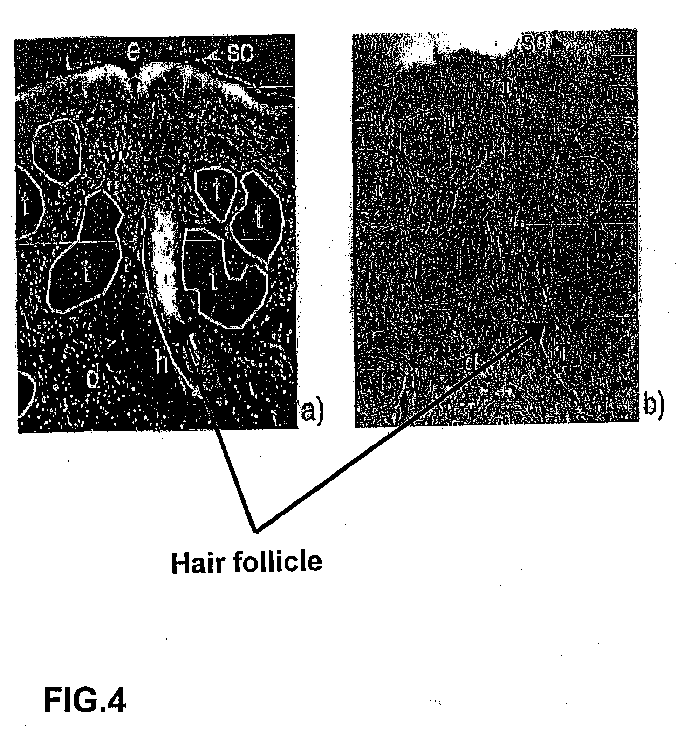

Fig. 4 shows a sample exhibiting the presence of PpIX in the pilo-sebaceous apparatus. The selective generation of PpIX in the hair follicle is clearly visible due to the bright fluorescence which appears white in the image a). The image b) is the corresponding hematoxylin-eosin stained image.

Semi-quantitative PpIX fluorescence intensities in different tissues obtained after microscopic analysis of 26 excised tumoral skin samples are summarized in table 1. An unexpectedly higher fluorescence is observed in the pilo-sebaceous apparatus after administration of n-hexyl-ALA, if compared to fluorescence observed in follicles after administration of ALA.

Iontophoretic transport of ALA, ALA-Me, ALA-Et, ALA-Bu, ALA-Hex and ALA-Oct was followed in vitro for two hours at constant current (0.5 mA/cm²), using dermatomed skin from the porcine ear (∼ 700 µm). Flow-through diffusion cells and Ag/AgCl electrodes were used. The anode formulations were 15 mM ALA or ALA ester (as the HCl salt) in water to which a minimum of NaOH was added to adjust the pH to ∼ 7. To ensure the presence of sufficient chloride in the donor compartment, the entire anodal formulation was removed and replaced after one hour with fresh 15 mM drug. The cathodal and receptor chambers of the diffusion cell contained physiological saline buffered with 25 mM HEPES to pH 7.4. The receptor was perfused continuously at 2 mL/h, and samples were collected automatically each 30 minutes and analyzed for transported drug by HPLC with fluorescence detection.

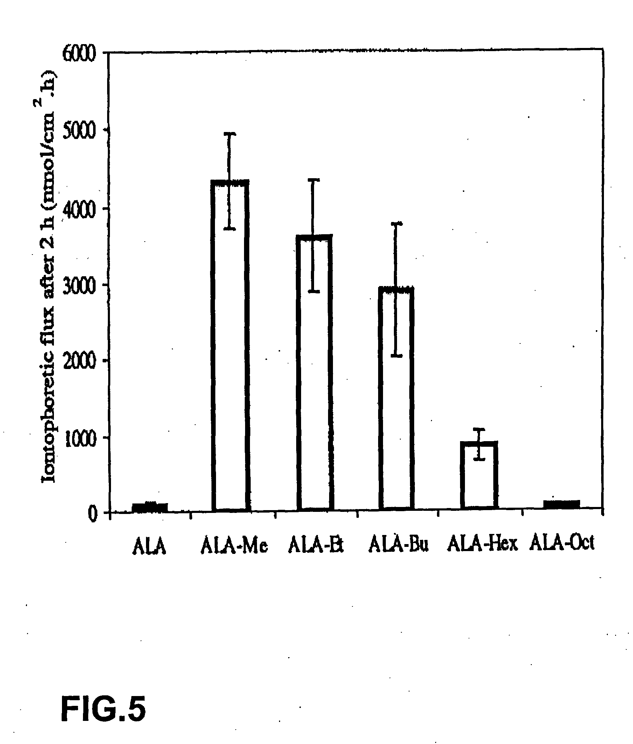

Fig. 5 presents the anodal iontophoretic transport of ALA and its methyl through octyl esters, after two hours, from a 15 mM solution of the drug, pH ∼ 7. Iontophoresis of the positively charged ALA-Me is enormously enhanced over that of the zwitterionic ALA. As the molecular weight and the lipophilicity of the esters increased, their transport progressively decreased.

Clearly, conversion of the net uncharged ALA to the short chain esters resulted in a small, rather mobile, cation which (there being essentially no competing positively charged species in the anode formulation) was able to transport by electromigration a significant fraction of the total charge passed between the electrodes. The transport number of ALA-Me, in fact, was greater than 0.2.

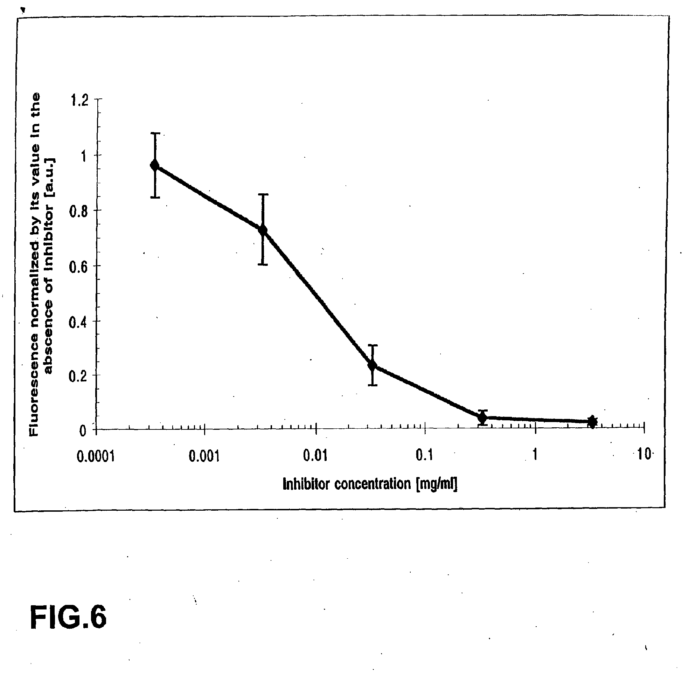

Fig. 6 illustrates the reduction of the PpIX production in T24 cells for different concentrations of an inhibitor of protoporphyrinogen oxidase ranging from 0.33 µg/ml to 3.3 mg/ml. The concentration of the ALA-n-hexylester was 0.1 mM. This decrease of PpIX production is unexpected considering the industrial use of these inhibitors as well as publications describing their ability to increase the PpIX level.

The above measurements illustrate an unexpected target selectivity of ALA-esters for hair follicles and an unexpected transdermal transport ability if compared to underivatised ALA, demonstrating that the use of these compounds in a hair removal method by photoinactivation is an advantageous alternative to the use of ALA itself. Nevertheless, those skilled in the art will appreciate that improved delivery techniques, improved irradiation techniques and/or improved formulations or kits incorporating agents and additives as disclosed above may also enhance efficiency of a hair removal method based on the use of ALA itself.

Moreover, the above measurements illustrate unexpected possibilities of modulation of PpIX precursors effects through the skin.

**Table 1:**

| PpIX fluorescence intensity of different skin components induced by the application of n-hexyl-ALA or ALA 0 no fluorescence, + week, ++ medium, +++ strong | | | | | | | | | | |
|---|---|---|---|---|---|---|---|---|---|---|
| No | precursor | time | sex | location | typ | epidermis | tumor | inflamn | hair | gland |
| 1 | 1% h-ALA | 2h20 | f | head | BCC | ++ | ++ | | | |
| 2 | | 2h20 | f | head | BCC | + | + | | ++ | +++ |
| 3 | | 3h | m | thorax | BCC | +++ | +++ | + | +++ | +++ |
| 4 | | 3h30 | m | head/neck | BCC | + | ++ | | +++ | |
| 5 | | 3h30 | m | thorax | BCC | + | + | | | |
| 6 | | 4h | f | head | BCC | ++ | ++ | | +++ | |
| 7 | | 4h | m | thorax | BCC | + | + | | +++ | |
| 8 | | 6h | f | head | BCC | +++ | +++ | | | |
| 9 | | 15h | m | neck | SCC | + | + | | ++ | ++ |
| 10 | | 15h | m | neck | BCC | 0 | ++ | | | |
| 11 | | 15h | m | thorax | BCC | ++ | ++ | | | |
| 12 | | 17h | f | head | BCC | +++ | +++ | | | |
| 13 | | 20h | f | head/neck | BCC | ++ | + | | ++ | |
| 14 | | 20h | f | head | BCC | 0 | 0 | | ++ | |
| 15 | | 24h | m | thorax | BCC | ++ | ++ | | ++ | |
| 16 | 20%A LA | 2h30 | m | head | SCC | + | + | | | |
| 17 | | 4h | f | foot | SCC | + | ++ | ++ | | |
| 18 | | 18h | m | shoulder | BCC | ++ | + | | | |
| 19 | | 18h | m | shoulder | BCC | + | + | | | |
| 20 | | 18h | m | shoulder | BCC | ++ | + | | | |
| 21 | | 18h | m | shoulder | BCC | ++ | + | + | | |
| 22 | | 20h | m | head | BCC | ++ | ++ | | +++ | +++ |
| 23 | | 20h | m | head | BCC | +++ | ++ | | +++ | +++ |
| 24 | | 20h | m | thorax | BCC | ++ | +++ | ++ | | |
| 25 | | 21h | m | head/neck | BCC | ++ | ++ | | | |
| 26 | | 24h | m | thorax | BCC | ++ | ++ | ++ | | +++ |

## Claims

1. Use of a compound of formula (I):
R²R³N-CH₂COCH₂-CH₂COOR¹ (I)
wherein R¹, R², R³ each separately represent H, an unsubstituted alkyl, or a substituted alkyl, wherein substitutents are selected from aryl, acyl, halogen, hydroxy, amino, aminoalkyl, alkoxy, acylamino, thioamino, acyloxy, aryloxy, aryloxyalkyl, mercapto, thia, azo, oxo groups, saturated and non-saturated cyclohydrocarbons, and heterocycles, hydroxy, alkoxy, acyloxy, amino, aryl, oxo or fluoro groups, or represent an alkyl chain interrupted by oxygen, nitrogen, sulfur or phosphor atoms, or an alkoxycarbonyloxy, alkoxycarbonylalkyl or methine group, and wherein R¹, R² and R³ do not represent simultaneously H, in a cosmetic method for hair removal from an area of skin of a mammal.

2. A use according to claim 1, **characterized in that** said compound of formula (I) is an ALA-ester or a pharmaceutically acceptable salt thereof, wherein R² and R³ both represent H and R¹ represents an alkyl from C1 to C12.

3. A use according to claim 2, wherein said compound of formula I is ALA-n-hexylester or ALA-n-octylester.

4. A method of removing hair from an area of skin of a mammal, using a compound of formula I according to anyone of claims 1, 2, 3, **characterized in that** a composition of matter comprising said compound is administered topically to said skin area.

5. A method according to claim 4, **characterized in that** said topical administration is accomplished by transdermal iontophoretic delivery.

6. A method according to claim 4 or 5, **characterized in that** cooling means are applied to said skin area.

7. A method according to anyone of claims 4 to 6, **characterized in that** the medium surrounding said area of skin is maintained free of oxygen.

8. A method as claimed in anyone of claims 4 to 7, **characterized in that** it comprises a preliminary step of synchronization of biological activity of hair follicles of said skin area.

9. A method as claimed in claim 8, **characterized in that** said synchronization step comprises the administration of minoxidyl.

10. A method according to anyone of claims 4 to 9, comprising a step of preliminary removal of the stratum corneum, or a preliminary treatment by heat and humidity.

11. A method for removing hair from an area of skin of a mammal, using a composition comprising a compound of formula I,
R²R³N-CH₂COCH₂-CH₂COOR¹ (I)
wherein R¹, R², R³ each separately represent H, an unsubstituted-alkyl, or a substituted alkyl, wherein substitutents are selected from aryl, acyl, halogen, hydroxy, amino, aminoalkyl, alkoxy, acylamino, thioamino, acyloxy, aryloxy, aryloxyalkyl, mercapto, thia, azo, oxo groups, saturated and non-saturated cyclohydrocarbons, and heterocycles, hydroxy, alkoxy, acyloxy, amino, aryl, oxo or fluoro groups, or represent an alkyl chain interrupted by oxygen, nitrogen, sulfur or phosphor atoms, or an alkoxycarbonyloxy or methine group, **characterized in that** said composition further comprises at least one agent selected from antioxidants, free radical scavengers and substances reacting with singlet oxygen.

12. A method as claimed in anyone of claims 4 or 11, **characterized in that** said composition comprises an agent reducing the PpIX level in the epidermis.

13. A method as claimed in anyone of claims 4 or 11, **characterized in that** said composition comprises at least one agent enhancing the local in vivo transformation of PpIX to heme in the epidermis.

14. A method as claimed in claim 13, **characterized in that** said composition comprises a protoporphyrinogen oxidase inhibitor.

15. A method as claimed in claim 13, **characterized in that** said composition comprises an agent inhibiting cellular transport of said compound of formula I in the epithelium.

16. A method as claimed in anyone of claims 11 to 15, **characterized in that** said composition further comprises an agent selected from agents enhancing penetration ability of said compound of formula I in the pilo-sebaceous apparatus.

17. A method according to anyone of claims 4 to 16, comprising at least a first irradiation of said skin area at a wavelength shorter than 600 nm, at a high light flux, in particular at light fluxes of between 50 - 150 mW/cm², followed by at least one irradiation at wavelengths longer than 600 nm, at a lower light flux.
